# EUROPÄISCHE PATENTSCHRIFT

(11) **EP 0 671 900 B1**
(45) Veröffentlichungstag und Bekanntmachung des Hinweises auf die Patenterteilung: **14.08.1996**
(21) Anmeldenummer: 94901871.7
(22) Anmeldetag: 25.11.1993
(51) Int. Cl.: A61K 7/13, C09B 51/00, C07C 211/48

(54) **ALLYLAMINO-NITROAROMATEN**
ALLYLAMINO-NITROAROMATES
ALLYLAMINO-NITROAROMATES

(30) Priorität: 03.12.1992 DE 4240684
(43) Veröffentlichungstag der Anmeldung: 20.09.1995
(73) Patentinhaber: Henkel Kommanditgesellschaft auf Aktien, 40191 Düsseldorf (DE)
(72) Erfinder: ROSE, David, D-40723 Hilden (DE); HÖFFKES, Horst, D-40595 Düsseldorf (DE); LIESKE, Edgar, Verstorben (DE); MATZIK, Iduna, D-40699 Erkrath (DE)
(86) Internationale Anmeldenummer: EP9303303
(87) Internationale Veröffentlichungsnummer: WO9412149

(56) Entgegenhaltungen:
- EP-A- 0 353 130
- EP-A- 0 378 463
- DE-A- 3 425 151
- DE-B- 1 924 249

## Beschreibung

Gegenstand der Erfindung sind neue Allylamino-nitroaromaten-Farbstoffe zum Färben von Fasern natürlichen Ursprungs und synthetischen Fasern.

In der Färberei spielen die direktziehenden Farbstoffe eine herausragende Rolle. Im Sinne der vorliegenden Erfindung ist ein direktziehender Farbstoff ein Farbstoff, der aus einem für die jeweils zu erzielende Färbung geeigneten Medium direkt auf die Faser aufzieht.

Nitroaromaten, die mit mindestens einer weiteren Alkyl- oder Hydroxyalkylaminogruppe substituiert sind, bilden eine wichtige Gruppe innerhalb der direktziehenden Farbstoffe. Solche Nitroaromaten sind z.B. in der Patentschrift DE 12 99 002, den deutschen Offenlegungsschriften DE 34 25 151 und DE 15 69 808 sowie der deutschen Auslegeschrift DE 11 70 583 beschrieben worden.

Es sind jedoch nur wenige Nitroaromaten mit einer zusätzlichen Allylaminogruppe bekannt. Die vorliegende Erfindung betrifft neue Allylamino-nitroaromaten, die sich hervorragend zum Färben von Fasern natürlichen Ursprungs und synthetischen Fasern eignen.

Gegenstand der Erfindung sind Allylamino-nitroaromaten der Formel I 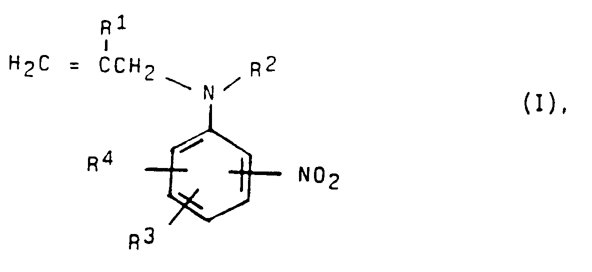 wobei R¹ Wasserstoff oder eine Alkylgruppe mit 1 bis 4 C-Atomen, R² Wasserstoff, eine Alkylgruppe mit 1 bis 4 C-Atomen oder eine Allylgruppe CH₂C(R⁵)=CH₂ darstellt, wobei R⁵ für Wasserstoff oder eine Alkylgruppe mit 1 bis 4 C-Atomen steht, R³ Carboxyl, Hydroxy, Nitro, Halogen oder eine Gruppe NR⁶R⁷ bedeutet, worin R⁶ und R⁷ unabhängig voneinander Wasserstoff, eine Alkylgruppe mit 1 bis 4 C-Atomen, eine Hydroxy- oder Dihydroxyalkylgruppe mit 2 bis 4 C-Atomen oder eine Allylgruppe CH₂C(R⁸)=CH₂ bedeuten, wobei R⁸ für Wasserstoff oder eine Alkylgruppe mit 1 bis 4 C-Atomen steht und R⁴ Wasserstoff oder Nitro darstellt, mit der Maßgabe, daß R³ und R⁴ nicht gleichzeitig Nitro bedeuten.

Ein bevorzugter Erfindungsgegenstand sind Allylamino-nitroaromaten der Formel I, in denen die NO₂-Gruppe in para-Position zur Allylamino-Gruppe N(R²)CH₂C(R¹)=CH₂ steht, R³ gleich Hydroxy ist und R⁴ Wasserstoff bedeutet.

Ein weiterer bevorzugter Erfindungsgegenstand sind Allylamino-nitroaromaten der Formel I, in denen die NO₂-Gruppe in ortho-Position zur Allylamino-Gruppe N(R²)CH₂C(R¹)=CH₂ steht, R³ gleich NR⁶R⁷ ist und in para-Position zur Allylamino-Gruppe N(R²)CH₂C(R¹)=CH₂ steht und R⁴ Wasserstoff bedeutet. Besonders bevorzugt sind dabei diejenigen Allylamino-nitroaromaten der Formel I, in denen R¹ und R² Wasserstoffe darstellen und R⁶ und R⁷ identische Gruppen sind. Ganz besonders bevorzugt ist 3-Nitro-4-allylamino-aminobenzol.

Ein weiterer Erfindungsgegenstand ist die Verwendung von Allylamino-nitroaromaten der Formel I zum Färben von Fasern natürlichen Ursprungs und synthetischen Fasern.

Unter Fasern natürlichen Ursprungs sind menschliche, tierische und pflanzliche Fasern wie z.B. Seide, Baumwolle, Leinen, Jute und Sisal und Keratinfasern wie Haare, Pelze, Wolle oder Federn aber auch regenerierte oder modifizierte Naturfasern wie z.B. Viskose, Nitro- und Acetylcellulose, Alkyl-, Hydroxylalkyl- und Carboxyalkylcellulosen zu verstehen. Aus der Klasse der synthetischen Fasern sind z.B. Polyamid-, Polyester-, Polyacrylnitril- und Polyurethanfasern zu nennen.

Die Verbindungen der Formel I können sowohl selbst als auch in Form ihrer wasserlöslichen Salze eingesetzt werden. Unter wasserlöslichen Salze sind Salze wie z.B. die Hydrochloride oder Hydrobromide zu verstehen. Es ist nicht erforderlich, daß eine einheitliche Verbindung der Formel I verwendet wird, vielmehr kann auch eine Mischung verschiedener Verbindungen der Formel I zum Einsatz kommen. Die Farbstoffe der Formel I erzeugen Färbungen in einem weiten Bereich von orangebraun bis blauviolett, wobei ihr Aufziehvermögen auf die Faser und ihr Egalisierverhalten überdurchschnittlich gut sind. Die erzielten Färbungen zeichnen sich außerdem durch sehr gute Licht-, Schweiß- und Waschechtheit aus.

Das Färben von Textilfasern geschieht vorzugsweise nach dem Aufziehverfahren bei Temperaturen über 90°C, andere für das Färben von Textilfasern übliche Färbeverfahren sind jedoch ebenfalls geeignet.

Bevorzugt werden die Allylamino-nitroaromaten der Formel I jedoch zum Färben von Keratinfasern, insbesondere menschlichen Haaren verwendet, da sie bereits bei physiologisch verträglichen Temperaturen unterhalb 40°C auf Keratinfasern bzw. menschliches Haar aufziehen und dieses intensiv einfärben. Es ist zu beachten, daß in der Haarfärberei an die Farbstoffe und an die erzielten Färbungen besondere Anforderungen gestellt werden. Die Farbstoffe müssen dermatologisch und toxikologisch unbedenklich sein, bei niedrigen Temperaturen auf die Haare aufziehen und gleichzeitig ein gutes Egalisierverhalten zeigen. Die Färbungen müssen gegen Haarbehandlungsmethoden, wie z.B. Dauerwellen, beständig sein. Diesen hohen Anforderungen werden die Allylamino-nitroaromaten der Formel I und die mit ihnen erzielten Färbungen in besonderem Maße gerecht.

Ein weiterer Patentgegenstand sind Haarfärbemittel enthaltend Farbstoffe der Formel I nach Anspruch 1 bis 3 in einer Menge von 0,01 bis 5 Gew.-%, vorzugsweise 0,1 bis 2 Gew.-%, bezogen auf das gesamte Haarfärbemittel, und einen wasserhaltigen kosmetischen Träger.

Wasserhaltige kosmetische Träger sind z.B. Cremes, Emulsionen, Gele oder auch tensidhaltige schäumende Lösungen, z.B. in Shampoos, Schaumerosolen oder andere Zubereitungen, die für die Anwendung auf dem Haar geeignet sind.

Übliche Bestandteile solcher wasserhaltiger kosmetischer Zubereitungen sind z. B. Netz- und Emulgiermittel, wie anionische, nichtionische und ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäure und Alkylphenole, Sorbitanfettsäureester und Fettsäurepartialglyceride, Fettsäurealkanolaminde sowie Verdickungsmittel, wie z. B. Methyl- oder Hydroxycellulose, Stärke, Fettalkohole, Paraffinöle, Fettsäuren, ferner Parfumöle und haarpflegende Zusätze, wie z. B. wasserlösliche kationische ampholytische und anionische Polymere, Proteinderivate, Pantothensäure und Cholesterin. Die Bestandteile der kosmetischen Träger werden zur Herstellung der erfindungsgemäßen Färbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Zur Modifikation der Färbungen können den Allylamino-nitroaromaten der Formel I weitere übliche direktziehende Farbstoffe, wie z.B. andere Nitrobenzolderivate, Antrachinonfarbstoffe, Triphenylmethan- oder Azofarbstoffe oder aber auch übliche Oxidationshaarfarbstoffvorprodukte zugemischt werden, bei denen zwischen Entwicklerkomponenten und Kupplerkomponenten zu unterscheiden ist. Entwicklerkomponenten bilden durch oxidative Kupplung untereinander oder gegebenenfalls in Gegenwart geeigneter Kupplerkomponenten die Oxidationshaarfarbstoffe aus. Als Entwicklersubstanzen werden z.B. primäre aromatische Amine mit einer weiteren in Para- oder Ortho-Position befindlichen freien oder substituierten Hydroxy- oder Aminogruppe, Diaminopyridinderivate, heterocyclische Hydrazone, 4-Aminopyrazolonderivate und 2, 4, 5, 6-Tetraaminopyrimidin und dessen Derivate eingesetzt. Als Kupplersubstanzen werden Metaphenylendiaminderivate, Naphthole. Resorcin und Resorcinderivate, Pyrazolone und Metaaminophenole verwendet.

Weitere direktziehende Farbstoffe und Oxidationshaarfarbstoffvorprodukte können in einer Menge von 0,01 bis 5 Gew.-%, vorzugsweise von 1 bis 3 Gew.-%, bezogen auf das gesamte Haarfärbemittel, enthalten sein.

Die Anwendung der erfindungsgemäßen Haarfärbemittel kann unabhängig von der Art der kosmetischen Zubereitung z. B. als Creme, Gel oder Shampoo im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

### Beispiele

### Herstellungsbeispiele

1. **3-Nitro-4-allylamino-aminobenzol**
   Eine Mischung bestehend aus 4-Fluor-3-nitroaminobenzol (7,8 g, 0,05 mol) und Allylamin (7,1 g, 0,125 mol) wurde 8 Stunden lang unter Rückfluß erhitzt. Nach dem Abkühlen wurde das Produkt abgesaugt und aus Ethanol/Wasser umkristallisiert.
   Dunkelrote Kristalle, Schmelzpunkt 80 - 83°C.
2. **3-Nitro-4-allylamino-N,N-bis-(2-hydroxyethyl)aminobenzol**
   Synthese und Aufarbeitung analog zu Verbindung 1, aber mit 4-Fluor-3-nitro-N,N-bis-(2-hydroxyethyl)-aminobenzol statt 4-Fluor-3-nitroaminobenzol.
   Schwarze Kristalle, Schmelzpunkt 73 - 75°C.
3. **3-Nitro-4-allylamino-N,N-bis-(2,3-dihydroxypropyl)-aminobenzol**
   Synthese analog zu Verbindung 1, aber mit 4-Fluor-3-nitro-N,N-bis-(2,3-dihydroxypropyl)-aminobenzol statt 4-fluor-3-nitro-aminobenzol.
   Aufarbeitung: Die Reaktionsmischung wurde eingeengt und das dunkle Öl in Ethanol gelöst. Verbindung (3) wurde anschließend mit HCl-Gas als Hydrochlorid gefällt.
   Gelbe Kristalle, Schmelzpunkt 154 - 158°C.
4. **3-Nitro-4-diallylamino-N-(2-hydroxyethyl)-aminobenzol**
   Eine Mischung bestehend aus 1-(2-Hydroxyethyl)-amino-2-nitro-4-aminobenzol (9,8 g, 0,05 mol), Allylbromid (6,1 g, 0,05 mol) und Kaliumcarbonat (3,5 g, 0,025 mol) in 80 ml Ethanol wurde 6 Stunden lang unter Rückfluß gekocht. Nach dem Abkühlen wurde abgesaugt und der Rückstand mit Ether extrahiert. Durch Einleitung von HCl-Gas wurde das Produkt als Hydrochlorid gefällt und anschließend abgesaugt.
   Gelbe Kristalle, Schmelzpunkt 166 - 170°C.
5. **3-Nitro-4-allylamino-N,N-bis-(allyl)-aminobenzol**
   Eine Mischung bestehend aus N-(2-Nitro-4-aminophenyl)-allylamin (1,9 g, 0,01 mol), Allylbromid (2,4 g, 0,02 mol) und Kaliumcarbonat (2,8 g, 0,02 mol) in 20 ml Ethanol wurde 7 Stunden lang unter Rückfluß gekocht. Die Aufarbeitung erfolgte analog zu Verbindung 4.
   Gelbe Kristalle, Schmelzpunkt 131 - 140°C.
6. **3-Nitro-4-(2-methylallyl)-amino-aminobenzol**
   Eine Mischung bestehend aus 4-Fluor-3-nitroaminobenzol (7,8 g, 0,05 mmol) und (2-Methylallyl)amin-hydrochlorid (0,05 mmol) in 100 ml Ethanol wurde 8 Stunden lang unter Rückfluß gekocht. Das Reaktionsprodukt wurde anschließend abgesaugt.
   Gelbe Kristalle.
7. **3-Nitro-4-allylamino-benoesäure**
   Synthese und Aufarbeitung analog zu Verbindung 1, aber mit 4-Chlor-3-nitro-benzoesäure statt 4-Fluor-3-nitroaminobenzol. Gelbe Kristalle, Schmelzpunkt 195 - 200°.

### Anwendungsbeispiele

### Färben von Textilfasern:

Zum Färben der Textilfasern wurde ein Mehrfaserbegleitgewebe (Multi-fiberfabric # 1, Loeffer Textilien, Nettersheim) verwendet. Ein solches Gewebe besteht aus 6 Textilstreifen, bestehend aus Polyacrylnitril, Acetylcellulose (Triacetat), Polyamid, Seide, Viskoseacetat und Wolle.

0,5 g des Farbstoffs (Verbindung 1, 2 oder 4 bis 7), 8 g Natriumsulfat-decahydrat und 2 g Natriumcarbonat wurden bei 60 °C in 100 ml Wasser gelöst. Dem Färbebad wurde das Mehrfaserbegleitgewebe zugegeben. Anschließend wurde innerhalb von 45 Minuten auf eine Temperatur von 98 °C erhöht. Diese Temperatur wurde 1 Stunde lang beibehalten, wobei das verdampfte Wasser kontinuierlich ersetzt wurde. Danach wurde das gefärbte Gewebe zunächst mit kaltem und anschließend mit heißem Nasser gespült. Das Gewebe wurde anschließend 20 Minuten lang in 250 ml 0,25 g Natriumlaurylsulfat enthaltendem Wasser gekocht und anschließend gespült. Zum Schluß wurde das Gewebe getrocknet. Die Färbeergebnisse sind Tabelle 1 zu entnehmen.

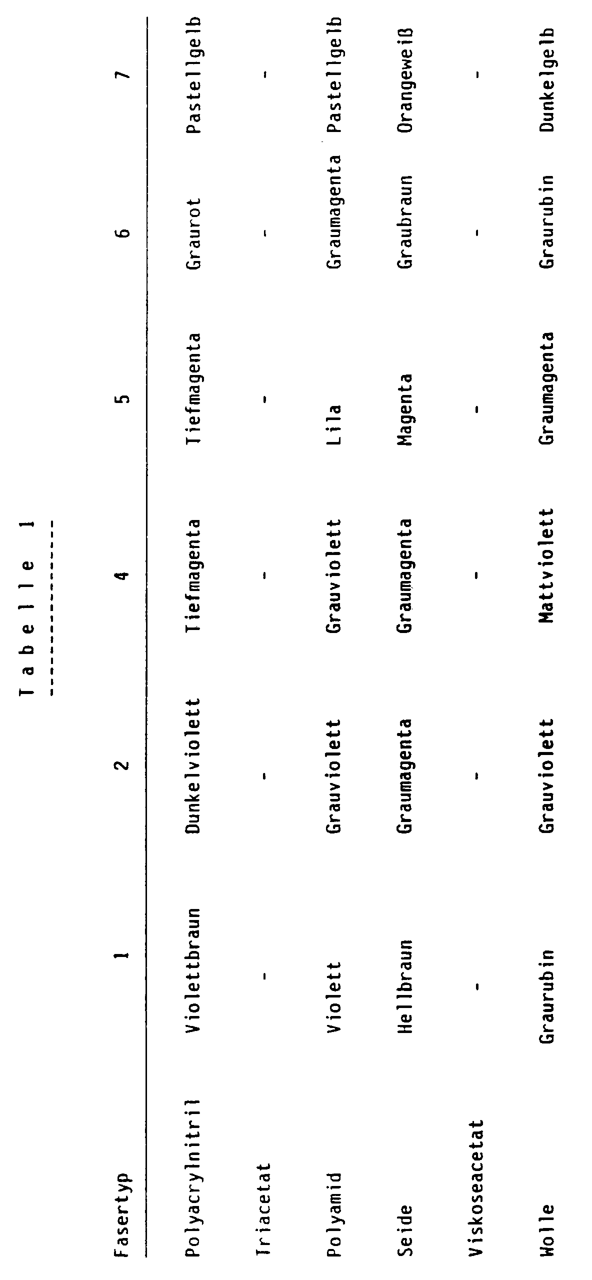

### Färben von menschlichen Haaren

Die Verbindungen 8, 9 und 10 sind literaturbekannte direktziehende Farbstoffe, die zum Vergleich herangezogen werden:
(8) 3-Nitro-4-(2-hydroxyethyl)amino-aminobenzol
(9) 2-Nitro-p-phenylendiamin
(10) 3-Nitro-4-(2-hydroxyethyl)amino-N,N-bis(2-hydroxyethyl)aminobenzol

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄ + 2 EO Sulfat, Na-Salz (28 %ig) | 25 g |
| Wasser | 60 g |
| Verbindung 1 - 3, 7 - 10 | 1 g |
| Ammoniumsulfat | 1 g |
| konz. Ammoniaklösung | bis pH = 9,5 |
| Wasser | ad 100 g |

Die Bestandteile wurden der Reihe nach miteinander vermischt. Nach Zugabe der Verbindungen 1, 2, 3, 7, 8, 9 oder 10 wurde mit konz. Ammoniaklösung der pH-Wert der Emulsion auf 9,5 eingestellt, dann wurde mit Wasser auf 100 g aufgefüllt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Das Ergebnis der Färbeversuche ist Tabelle 2 zu entnehmen.

Tabelle 2 enthält weiterhin die Gesamtfarbabstands-Werte einer jeden Färbung; der Gesamtfarbabstand wird an Egalisiersträhnen gemessen und gibt Informationen über das Egalisierverhalten des Farbstoffes.

Egalisiersträhnen stellt man her, indem man die obere Hälfte einer Haarsträhne (der Bereich der Haarspitze) 30 Minuten lang mit der wäßrigen Lösung eines Kaltwellmittels auf Basis Ammoniumthioglykolat behandelt. Nach der Fixierung (10 Minuten, Kaliumbromat-Lösung) wird die Strähnenhälfte gewaschen und getrocknet. Dann wird sie mit einer wäßrigen Lösung aus Wasserstoffperoxid und Ammoniumperoxiddisulfat ultrablondiert. Anschließend erfolgt noch einmal eine Behandlung mit dem Kaltwellmittel, der Fixierung und der Ultrablondierung. Die untere Hälfte der Haarsträhne (Wurzelbereich) wird nur einmal ultrablondiert. Auf diese Weise werden 2 unterschiedlich stark strapazierte Haarsträhnen-Hälften erhalten.

Die Egalisierung wird in DE-Werten (Gesamt-Farbabstand zwischen den beiden Enden der Egalisiersträhne) angegeben. Die DE-Werte werden wie folgt ermittelt:
Jede Egalisier-Haarsträhne wird an acht Stellen (4 im Bereich des Haaransatzes und 4 im Bereich der Haarspitze) mit Hilfe eines Farbmeßsystems der Firma Datacolor vermessen. Dabei wird die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner (Minicomputer HP 2113 E) verarbeitet. Das Rechnerprogramm ermittelt die Normfarbwerte nach dem CIE-System (Commission Internationale de l'Eclairage) entsprechend DIN 5033 und rechnet sie in Farbabstandszahlen nach DIN 6174 um.

**Tabelle 2**

| Verbindung | Nuance des gefärbten Haares | Gesamt-Farbabstand (DE-Wert) |
|---|---|---|
| 1 | Violettbraun | 3,7 |
| 2 | Blauviolett | 7,4 |
| 3 | Purpur | 4,1 |
| 7 | Dunkelolivgelb | 6,1 |
| 8 | Purpurrot | 12,56 |
| 9 | Orangerot | 19,9 |
| 10 | Rotviolett | 15,75 |

## Patentansprüche

1. Allylamino-nitroaromaten der Formel I 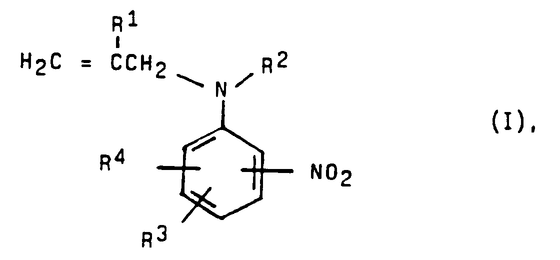 wobei R¹ Wasserstoff oder eine Alkylgruppe mit 1 bis 4 C-Atomen, R² Wasserstoff, eine Alkylgruppe mit 1 bis 4 C-Atomen oder eine Allylgruppe CH₂C(R⁵)=CH₂ darstellt, wobei R⁵ für Wasserstoff oder eine Alkylgruppe mit 1 bis 4 C-Atomen steht, R³ Carboxyl, Hydroxy, Nitro, Halogen oder eine Gruppe NR⁶R⁷ bedeutet, worin R⁶ und R⁷ unabhängig voneinander Wasserstoff, eine Alkylgruppe mit 1 bis 4 C-Atomen, eine Hydroxy- oder Dihydroxyalkylgruppe mit 2 bis 4 C-Atomen oder eine Allylgruppe CH₂C(R⁸)=CH₂ bedeuten, wobei R⁸ für Wasserstoff oder eine Alkylgruppe mit 1 bis 4 C-Atomen steht und R⁴ Wasserstoff oder Nitro darstellt, mit der Maßgabe, daß R³ und R⁴ nicht gleichzeitig Nitro bedeuten.

2. Allylamino-nitroaromaten der Formel I, wobei die NO₂-Gruppe in para-Position zur Allylamino-Gruppe N(R²)CH₂C(R¹)=CH₂ steht, R³ gleich Hydroxy ist und R⁴ Wasserstoff bedeutet.

3. Allylamino-nitroaromaten der Formel I, wobei die NO₂-Gruppe in ortho-Position zur Allylamino-Gruppe N(R²)CH₂C(R¹)=CH₂ steht, R³ gleich NR⁶R⁷ ist und in para-Position zur Allylamino-Gruppe N(R²)CH₂C(R¹)=CH₂ steht und R⁴ Wasserstoff bedeutet.

4. Allylamino-nitroaromaten der Formel I nach Anspruch 3, wobei R¹ und R² Wasserstoffe darstellen und R⁶ und R⁷ identische Gruppen sind; vorzugsweise 3-Nitro-4-allylamino-aminobenzol.

5. Verwendung von Allylamino-nitroaromaten der Formel I nach Anspruch 1 bis 4 zum Färben von Fasern natürlichen Ursprungs und synthetischen Fasern.

6. Verwendung von Allylamino-notroaromaten der Formel I nach Anspruch 1 bis 5 zum Färben von Keratinfasern, insbesondere menschlichen Haaren.

7. Haarfärbemittel enthaltend Verbindungen der Formel I nach Anspruch 1 bis 4 in einer Menge von 0,01 bis 5 Gew.-%, vorzugsweise 0,1 bis 2 Gew.-%, bezogen auf das gesamte Haarfärbemittel, und einen wasserhaltigen kosmetischen Träger.

## Claims

1. Aromatic allylaminonitro compounds corresponding to formula (I): 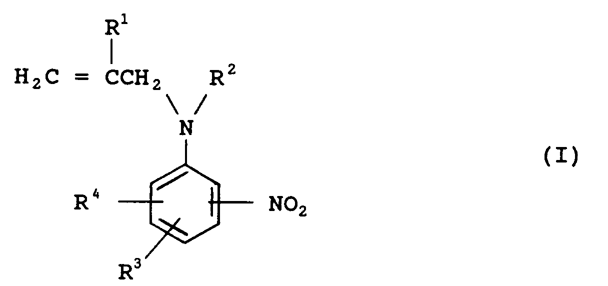 in which R¹ is hydrogen or an alkyl group containing 1 to 4 carbon atoms, R² is hydrogen, an alkyl group containing 1 to 4 carbon atoms or an allyl group CH₂C(R⁵)=CH₂, where R⁵ is hydrogen or an alkyl group containing 1 to 4 carbon atoms, R³ is carboxyl, hydroxy, nitro, halogen or a group NR⁶R⁷, where R⁶ and R⁷ independently of one another represent hydrogen, an alkyl group containing 1 to 4 carbon atoms, a hydroxyalkyl or dihydroxyalkyl group containing 2 to 4 carbon atoms or an allyl group CH₂C(R⁸)=CH₂, where R⁸ is hydrogen or an alkyl group containing 1 to 4 carbon atoms, and R⁴ is hydrogen or nitro, with the proviso that R³ and R⁴ are not both nitro.

2. Aromatic allylaminonitro compounds corresponding to formula I, in which the NO₂ group is in the para position to the allylamino group N(R²)CH₂C(R¹)=CH₂, R³ is hydroxy and R⁴ is hydrogen.

3. Aromatic allylaminonitro compounds corresponding to formula I, in which the NO₂ group is in the ortho position to the allylamino group N(R²)CH₂C(R¹)=CH₂, R³ represents NR⁶R⁷ and is in the para position to the allylamino group N(R²)CH₂C(R¹)=CH₂, and R⁴ is hydrogen.

4. Aromatic allylaminonitro compounds corresponding to formula I in claim 3, in which R¹ and R² are hydrogen and R⁶ and R⁷ are identical groups; preferably 3-nitro-4-allylamino-aminobenzene.

5. The use of the aromatic allylaminonitro compounds of formula I claimed in claims 1 to 4 for the dyeing of fibers of natural origin and synthetic fibers.

6. The use of the aromatic allylaminonitro compounds of formula I claimed in claims 1 to 4 for the dyeing of keratin fibers, more particularly human hair.

7. Hair dye formulations containing the compounds of formula I claimed in claims 1 to 4 in a quantity of 0.01 to 5% by weight and preferably in a quantity of 0.1 to 2% by weight, based on the hair dye formulation as a whole, and a water-containing cosmetic carrier.

## Revendications

1. Composés allylamino-nitroaromatiques de la formule I 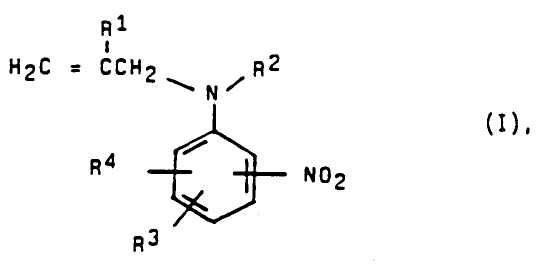 dans laquelle R¹ représente l'hydrogène ou un groupe alkyle comportant 1 à 4 atomes de C, R² correspond à l'hydrogène, à un groupe alkyle possédant 1 à 4 atomes de C ou à un groupe allyle CH₂C(R⁵)=CH₂, dans lequel R⁵ représente l'hydrogène ou un groupe alkyle comportant 1 a 4 atomes de C, R³ est un carboxyle, un hydroxy, un nitro, un halogène ou un groupe NR⁶R⁷, dans lequel R⁶ et R⁷ représentent indépendamment l'un de l'autre l'hydrogène, un groupe alkyle possédant 1 à 4 atomes de C, un groupe hydroxy- ou dihydroxyalkyle comportant 2 à 4 atomes de C ou un groupe allyle CH₂C(R⁸)=CH₂, dans lequel R⁸ représente l'hydrogène ou un groupe alkyle possédant 1 a 4 atomes de C, et R⁴ correspond a l'hydrogène ou à un nitro, à condition que R³ et R⁴ ne représentent pas simultanément un nitro.

2. Composés allylamino-nitroaromatiques de la formule I, dans lesquels le groupe NO₂ se trouve en position para par rapport au groupe allylamino N(R²)CH₂C(R¹)=CH₂, R³ est égal a un hydroxy et R⁴ correspond à l'hydrogène.

3. Composés allylamino-nitroaromatiques de la formule I, dans lesquels le groupe NO₂ se trouve en position ortho par rapport au groupe allylamino N(R²)CH₂C(R¹)=CH₂, R³ correspond à NR⁶R⁷ et se trouve en position para par rapport au groupe allylamino N(R²)CH₂C(R¹)=CH₂ et R⁴ correspond à l'hydrogène.

4. Composés allylamino-nitroaromatiques de la formule I, selon la revendication 3, dans lesquels R¹ et R² représentent de hydrogène, et R⁶ et R⁷ des groupes identiques; de préférence le 3-nitro-4-allylamino-aminobenzène.

5. Utilisation de composés allylamino-nitroaromatiques de la formule I selon les revendications 1 à 4, pour la teinture de fibres d'origine naturelle et synthétique.

6. Utilisation de composés allylamino-nitroaromatiques de la formule I selon les revendications 1 à 5, pour la teinture de fibres de kératine, en particulier de la chevelure humaine.

7. Teintures capillaires contenant les composés de la formule I, selon les revendications 1 à 4, dans une proportion de 0,01 à 5 % en poids, de préférence de 0,1 à 2 % en poids, par rapport à la totalité de la teinture capillaire, et un support cosmétique aqueux.
